# FASCICULE DE BREVET EUROPEEN

(11) **EP 3 764 977 B1**
(45) Date de publication et mention de la délivrance du brevet: **16.04.2025**
(21) Numéro de dépôt: 18800897.3
(22) Date de dépôt: 07.11.2018
(51) Int. Cl.: A61K 8/365, A61K 8/73, A61Q 19/10, C11D 17/00

(54) **FORMULATION COSMETIQUE**
KOSMETISCHE FORMULIERUNG
COSMETIC FORMULATION

(30) Priorité: 07.11.2017 FR 1760457
(43) Date de publication de la demande: 20.01.2021
(73) Titulaire: Beaute Prestige International, 75008 Paris (FR)
(72) Inventeur: DRAGO, Marie, London N16 7TY (GB)
(74) Mandataire: Tripoz, Inès
(86) Numéro de dépôt international: PCT/EP2018/080531
(87) Numéro de publication internationale: WO 2019/092060

(56) Documents cités:
- EP-A1- 1 829 956
- EP-B1- 1 829 956
- DATABASE GNPD [online] MINTEL; March 2016 (2016-03-01), "Soap-Free Wash Bar", XP002782477, Database accession no. 3871483
- DATABASE GNPD [online] MINTEL; February 2016 (2016-02-01), "Intolerant Skin Bar Soap", XP002782478, Database accession no. 3790799
- DATABASE GNPD [online] MINTEL; January 2012 (2012-01-01), "Dermopan Dermo Cleansing Bar Soap", XP002782479, Database accession no. 1717670
- DATABASE GNPD [online] MINTEL; August 2017 (2017-08-01), "Emulave Bar Soap", XP002782480, Database accession no. 5021683
- DATABASE GNPD [online] MINTEL; December 2008 (2008-12-01), "Soap-Free Cleansing Bar", XP002782481, Database accession no. 1020956

## Description

La présente invention se rapporte au domaine de l'hygiène et de la cosmétique, et plus précisément au domaine des formulations cosmétiques et nettoyantes solides.

L'invention se rapporte à une formulation cosmétique solide caractérisée en ce qu'elle comprend une base comprenant au moins un syndet, un prébiotique et un postbiotique.

Dans la cadre de la présente demande, un certain nombre de définitions doivent être données.

On entend par « solide » un objet caractérisé à l'échelle macroscopique par un volume et une forme déterminés, constants en l'absence de toute force extérieure ou condition extrême.

On entend par "pourcentage massique" une grandeur définie comme un pourcentage entre la masse d'un composé et la masse totale d'une formulation le comprenant. Par exemple, si 10 grammes d'un composé y sont présents dans une formulation z ayant une masse totale de 100 grammes, alors le pourcentage massique de y dans z est 10%.

On entend par "rapport massique" le rapport entre la masse de deux composés présents dans la même formulation. Par exemple, si une formulation comprend 3 grammes de composé f et 1 gramme de composé g, alors le rapport massique f/g est 3/1 soit 3.

On entend par « syndet » un surfactant ou tensio-actif d'origine synthétique ou hémisynthétique. Un syndet permet le nettoyage du tégument tout en possédant les propriétés nettoyantes des savons classiques (issus de sources organiques). Un des avantages de l'utilisation de syndet est la préservation de la faune et de la flore.

Il existe différents types de syndet, dont les surfactants ou tensio-actifs anioniques (comme par exemple le sodium lauryl sulfate, l'acyl isethionate, l'alkylsulfonsuccinate de sodium, le sodium cocoyl isethionate ou encore le disodium lauryl sulfosuccinate), les surfactants ou tensio-actifs cationiques (comme par exemple le benzalkonium chloride), les surfactants ou tensio-actifs amphotères (comme par exemple la cocamidopropyl betaine, le coco ampho acetate ou encore le diacétate), ou encore les surfactants ou tensio-actifs non ioniques, et leurs mélanges dans toutes les proportions.

On entend par « formulation de base comprenant au moins un syndet » une formulation de base utilisée pour la préparation d'une formulation cosmétique solide permettant le nettoyage du tégument, comprenant au moins un syndet tel que défini ci-dessus. Il est notable que la formulation de base peut comprendre également d'autres ingrédients, notamment des liants, des solvants, des agents absorbants, des agents de conditionnement de la peau, des agents de conditionnement capillaire, des stabilisateurs d'émulsion, des agents moussant, des émollients, des agents masquant, des régulateurs de pH, des opacifiants, ainsi que d'autres ingrédients cosmétiquement acceptables. Par exemple, en sus de l'au moins un syndet, la base peut comprendre une certaine quantité de savon (on entend par « savon » un composé comprenant des molécules amphiphiles obtenues par réaction chimique dite de saponification entre un corps gras et une base forte).

On entend par « prébiotique » un ingrédient alimentaire non-digestible qui affecte de façon bénéficiaire l'hôte en stimulant la croissance et/ou l'activité d'une ou d'un nombre limité de bactéries du colon. Cette définition de 1995 a été affinée en 2004 avec les trois caractéristiques suivantes :
- résistance à l'acidité gastrique, à l'hydrolyse par les enzymes des mammifères et l'absorption gastro-intestinale ;
- fermentation par la microflore intestinale ;
- stimulation sélective de la croissance et/ou de l'activité des bactéries intestinales associées à la santé et au bien-être.

Les prébiotiques sont très souvent des sucres, ils sont considérés comme des fibres non digestibles et on peut les classer en fonction de leur degré de polymérisation en monosaccharides, disaccharides ou oligosaccharides.
- Parmi les monosaccharides, on citera par exemple le Tagatose, qui est naturellement présent en petites quantités dans la nature, et qui peut être produit à partir du lactose par isomérisation du galactose. Surtout reconnu pour ses propriétés sucrantes et antidiabétiques, il est aussi dégradé par certaines bactéries de l'intestin de genre Enterococcus et Lactobacillus.
- Parmi les disaccharides, on citera le lactulose. Composé d'un fructose et d'un galactose, le lactulose est un produit naturel dérivé du traitement du lait par haute température. Les études montrent une activité prébiotique sur les bactéries de type Bifidus, mais il est commercialisé principalement pour ses qualités de laxatif osmotique.
- Parmi les oligosaccharides, le plus commun est l'inuline, un polymère d'oligofructose (abrégé en FOS pour Fructo-oligosaccharides) naturellement présent dans la nature. En fonction du degré de polymérisation on peut également parler d'oligofructose.

D'autres oligosaccharides peuvent être également être cités comme les transgalactooligosaccharides (TOS) qui sont constitués d'un mélange d'oligosaccharides dérivés du lactose par transglycosylation enzymatique. Leur composition précise dépend des saccharides d'origine et des enzymes utilisées. Généralement, ce sont des tri-, tétra- ou pentasaccharides comme le polydextrose, ou le galactooligosaccharides (GOS) qui sont présents naturellement par exemple dans le colostrum et le lait maternel.

Même si comme vu précédemment, les prébiotiques sont très souvent des sucres, il peut également s'agir de peptides, on parle alors de « prébiotique peptidique ». Il existe également des prébiotiques sous forme d'acides linoléiques conjugués, d'acides gras polyinsaturés, de composés phénoliques, ou encore de composés phytochimiques.

On entend par « postbiotique » un produit bactérien ou un métabolite d'organisme probiotique qui a une activité biologique sur l'hôte. L'acide lactique, l'acide acétique, l'acide butyrique, l'acide propionique, les produits de fermentation (produits résultants de la transformation de certaines matières organiques, comme par exemple des sucres, sous l'action d'enzymes sécrétées par des micro-organismes) et les acides gras à chaine courte (comprenant entre 1 à 6 atomes de carbones) peuvent être considérés comme des postbiotiques.

On entend par « zone d'information » une zone visible à la surface de la composition cosmétique solide et comprenant une information intelligible. Cette information peut être une combinaison de lettres et/ou de chiffres, et être réalisée en relief positif ou négatif.

On entend par « activité de l'eau » le rapport entre la pression partielle de l'eau dans le produit à une certaine température et la pression partielle de l'eau pure à la même température. La valeur de ce rapport varie de 0 à 1 sachant que 0 correspond à un produit sec, c'est-à-dire que toute l'eau est liée au produit et donc qu'aucune molécule d'eau dans le produit n'est réactive.

Dans le cadre de la présente demande, le pH de ladite formulation cosmétique solide selon l'invention est mesuré par une méthode interne consistant à réaliser une solution à 1% de la formulation cosmétique solide dilué dans de l'eau, la solution est chauffée afin de solubiliser la formulation cosmétique solide puis la solution est refroidie jusqu'à la température ambiante (20°C), le pH est ensuite mesuré sur cette solution à température ambiante (20°C).

Enfin, dans le cadre de la présente demande, lorsqu'il est fait référence à « au moins un composé X » en rapport avec des valeurs numériques et/ou des intervalles de quantité (concentration, etc.), lorsque plusieurs composés X sont présents, alors les valeurs numériques et/ou les intervalles de quantité s'appliquent à la somme des composés X. Par exemple, si la formulation cosmétique solide comprend deux prébiotiques différents, i la formulation sera caractérisée en ce qu'elle comprend entre 0,1 et 1 % dudit au moins un prébiotique, cela signifie que la somme des quantités de prébiotiques est comprise entre 0,1 et 1%.

L'Homme se distingue de son environnement, au niveau d'une interface environnement/tégument, ledit tégument étant constitué des phanères et de la peau.

La fonction essentielle du tégument est de former une barrière efficace entre l'intérieur et l'extérieur de l'organisme.

Le tégument se compose des phanères et de la peau, la peau constituant, chez l'Homme, l'organe le plus lourd du corps.

La peau a une fonction essentielle de protection de l'organisme vis-à-vis de l'environnement. Elle comprend trois couches principales, de l'extérieur vers l'intérieur : épiderme, derme, hypoderme.

La barrière formée par l'épiderme est de trois types : barrière physique, barrière immune, barrière microbienne.

La barrière microbienne, ou autrement appelée microbiote, est constituée notamment de bactéries commensales présentes sur la surface de la peau. Le microbiote protège contre les microorganismes pathogènes grâce à l'activité antimicrobienne directe des bactéries commensales et à leur capacité à induire des réponses immunes protectrices. La microflore cutanée est organisée en biofilm, qui doit se renouveler en permanence et joue un rôle réel de protection contre les infections. La microflore commensale cutanée peut prévenir un certain nombre de colonisations et d'infections par une grande variété de micro-organismes pathogènes. Alors que la flore normale de la peau constitue une défense pour l'hôte, une augmentation ou une réduction de la composition bactérienne (dysbiose) conduit à une altération de la barrière cutanée, une diminution de l'immunité et prédispose à l'apparition de maladies inflammatoires (dermatite atopique, acné, rosacée, psoriasis, etc.).

Dans la période historique récente, il est considéré comme normal de laver la peau au moyen de divers détergents de manière quotidienne. Cette pratique très répandue met à mal la barrière microbienne, qui doit s'adapter aux agressions. Il est donc nécessaire de disposer de formulations cosmétiques nettoyantes adaptées à l'environnement bactérien cutané.

L'acide lactique fait partie du groupe des alpha-hydroxy acides comme l'acide glycolique, l'acide citrique et l'acide malique. Produit naturellement par les muscles du corps humain, il est aussi synthétisé par certaines bactéries (bactéries à acide lactique). L'acide lactique étant une molécule chirale, celle-ci peut être sous deux formes et leurs mélanges en différentes proportions dont le mélange racémique : D (CAS 10326-41-7), L (CAS 79-33-4), ou DL (CAS 50-21-5). Les humains ne synthétisent que la forme L, tandis que les bactéries peuvent synthétiser les deux formes. En utilisation cosmétique, on note moins d'irritations avec la forme L.

L'acide acétique (CAS 64-19-7), aussi appelé acide éthanoïque, est un acide gras à chaine courte synthétisé par certaines bactéries. Il est notamment présent dans le vinaigre.

L'acide butyrique (CAS 107-92-6), aussi appelé acide butanoïque, est un acide gras à chaine courte synthétisé par certaines bactéries. Il est notamment présent dans le beurre et dans certains fromages.

L'acide propionique (CAS 79-09-4), aussi appelé acide propanoïque, est un acide gras à chaine courte synthétisé par certaines bactéries. L'acide propionique serait responsable des odeurs corporelles, c'est un produit de dégradation des acides aminés et des acides gras à chaine longue par les bactéries du tégument telle que *Propionibacterium.* L'acide lactique, l'acide acétique, l'acide butyrique, l'acide propionique et, de manière générale, les acides gras à chaine courte peuvent être considérés comme des postbiotiques.

Dans l'art antérieur, un certain nombre de compositions ont été décrites.

Dans la demande WO2006015726 ou encore dans la demande US20170087083 au nom de HENKEL, des formulations comprenant des syndets sont citées. Ceci est également le cas pour la demande EP1829956 au nom de SEBAPHARMA.

Les formulations cosmétiques solides présentent un certain nombre d'avantages (facilités de manipulation, bonne conservation, etc.).

Le but de l'invention est de proposer une formulation cosmétique comprenant au moins une formulation de base comprenant au moins un syndet, améliorée.

L'invention concerne une formulation cosmétique solide, caractérisée en ce qu'elle comprend une formulation de base comprenant au moins un syndet, au moins un prébiotique et au moins un postbiotique.

La formulation cosmétique solide procure un certain nombre d'effet techniques surprenants et inattendus.

Tout d'abord, la formulation cosmétique solide selon l'invention a de bonnes caractéristiques physiques (facilité de manipulation, douceur au toucher). Les caractéristiques physiques permettent d'inclure, à la surface de ladite formulation, une zone d'information qui s'avère être utile.

Par ailleurs, l'inclusion de syndet(s) présente des avantages certains, par rapport aux savons classiques. Les syndets respectent les peaux sensibles (respect de l'intégrité de la peau), et permettent également une moindre consommation de ressources naturelles (faune, flore).

Par ailleurs, il a été mis en évidence que de telles caractéristiques solides pouvaient être obtenues pour des formulations cosmétiques solides ayant des pH inhabituels, par exemples inférieurs à 6, et plus précisément compris entre 5 et 6, ce qui représente un défi technique (un abaissement de pH a tendance à influencer de manière négative les propriétés physiques, la formulation peut devenir collante, peu apte à la solidification et à accueillir une zone d'information).

Enfin, d'un point de vue cosmétique, la présence de prébiotique et de postbiotique permet un parfait respect de l'intégrité de la barrière microbienne. Par ailleurs le fait que le pH de la formulation cosmétique solide selon l'invention puisse être inférieur à 6 est également avantageux, dans la mesure où ce dernier est proche de celui de la peau et des muqueuses.

La formulation cosmétique solide selon l'invention est ainsi parfaitement adaptée à un usage quotidien, plébiscité dans les sociétés modernes et est particulièrement compatible avec les muqueuses, ceci permet un usage sur les parties intimes.

Ainsi, l'invention concerne une formulation cosmétique solide, caractérisée en ce qu'elle comprend :
- une formulation de base comprenant au moins un syndet ;
- au moins deux prébiotiques différents ;
- au moins deux postbiotiques différents dont l'acide lactique et l'arginine ;
et dont la fabrication ne comprend aucune étape de moulage.

Dans un mode de réalisation, la formulation cosmétique solide selon l'invention est caractérisée en ce que le pH de ladite formulation cosmétique solide cosmétique est inférieur à 6.

Dans un mode de réalisation, la formulation cosmétique solide selon l'invention est caractérisée en ce que le pH de ladite formulation cosmétique solide cosmétique est inférieur à 5,5.

Dans un mode de réalisation, la formulation cosmétique solide selon l'invention est caractérisée en ce que le pH de ladite formulation cosmétique solide cosmétique est compris entre 5 et 6.

Dans un mode de réalisation, la formulation cosmétique solide selon l'invention est caractérisée en ce qu'elle comprend moins de 5% d'eau, en pourcentage massique par rapport à la masse totale de ladite formulation cosmétique solide.

Dans un mode de réalisation, la formulation cosmétique solide selon l'invention est caractérisée en ce qu'elle comprend moins de 4% d'eau, en pourcentage massique par rapport à la masse totale de ladite formulation cosmétique solide. Dans un mode de réalisation, la formulation cosmétique solide selon l'invention est caractérisée en ce qu'elle comprend moins de 3% d'eau, en pourcentage massique par rapport à la masse totale de ladite formulation cosmétique solide. Dans un mode de réalisation, la formulation cosmétique solide selon l'invention est caractérisée en ce qu'elle comprend moins de 2% d'eau, en pourcentage massique par rapport à la masse totale de ladite formulation cosmétique solide. Dans un mode de réalisation, la formulation cosmétique solide selon l'invention est caractérisée en ce qu'elle comprend moins de 1% d'eau, en pourcentage massique par rapport à la masse totale de ladite formulation cosmétique solide. Dans un mode de réalisation, la formulation cosmétique solide selon l'invention est caractérisée en ce qu'elle comprend moins de 0,9% d'eau, en pourcentage massique par rapport à la masse totale de ladite formulation cosmétique solide. Dans un mode de réalisation, la formulation cosmétique solide selon l'invention est caractérisée en ce qu'elle comprend moins de 0,8% d'eau, en pourcentage massique par rapport à la masse totale de ladite formulation cosmétique solide. Dans un mode de réalisation, la formulation cosmétique solide selon l'invention est caractérisée en ce qu'elle comprend moins de 0,7% d'eau, en pourcentage massique par rapport à la masse totale de ladite formulation cosmétique solide. Dans un mode de réalisation, la formulation cosmétique solide selon l'invention est caractérisée en ce qu'elle comprend moins de 0,5% d'eau, en pourcentage massique par rapport à la masse totale de ladite formulation cosmétique solide. Dans un mode de réalisation, la formulation cosmétique solide selon l'invention est caractérisée en ce qu'elle comprend moins de 0,3% d'eau, en pourcentage massique par rapport à la masse totale de ladite formulation cosmétique solide. Dans un mode de réalisation, la formulation cosmétique solide selon l'invention est caractérisée en ce qu'elle comprend moins de 0,2% d'eau, en pourcentage massique par rapport à la masse totale de ladite formulation cosmétique solide.

Dans un mode de réalisation, la formulation cosmétique solide selon l'invention est caractérisée en ce qu'elle comprend moins de 5% d'eau à l'issue du procédé de fabrication, en pourcentage massique par rapport à la masse totale de ladite formulation cosmétique solide.

Dans un mode de réalisation, la formulation cosmétique solide selon l'invention est caractérisée en ce qu'elle comprend moins de 4% d'eau à l'issue du procédé de fabrication, en pourcentage massique par rapport à la masse totale de ladite formulation cosmétique solide. Dans un mode de réalisation, la formulation cosmétique solide selon l'invention est caractérisée en ce qu'elle comprend moins de 3% d'eau à l'issue du procédé de fabrication, en pourcentage massique par rapport à la masse totale de ladite formulation cosmétique solide. Dans un mode de réalisation, la formulation cosmétique solide selon l'invention est caractérisée en ce qu'elle comprend moins de 2% d'eau à l'issue du procédé de fabrication, en pourcentage massique par rapport à la masse totale de ladite formulation cosmétique solide. Dans un mode de réalisation, la formulation cosmétique solide selon l'invention est caractérisée en ce qu'elle comprend moins de 1% d'eau à l'issue du procédé de fabrication, en pourcentage massique par rapport à la masse totale de ladite formulation cosmétique solide. Dans un mode de réalisation, la formulation cosmétique solide selon l'invention est caractérisée en ce qu'elle comprend moins de 0,9% d'eau à l'issue du procédé de fabrication, en pourcentage massique par rapport à la masse totale de ladite formulation cosmétique solide. Dans un mode de réalisation, la formulation cosmétique solide selon l'invention est caractérisée en ce qu'elle comprend moins de 0,8% d'eau à l'issue du procédé de fabrication, en pourcentage massique par rapport à la masse totale de ladite formulation cosmétique solide. Dans un mode de réalisation, la formulation cosmétique solide selon l'invention est caractérisée en ce qu'elle comprend moins de 0,7% d'eau à l'issue du procédé de fabrication, en pourcentage massique par rapport à la masse totale de ladite formulation cosmétique solide. Dans un mode de réalisation, la formulation cosmétique solide selon l'invention est caractérisée en ce qu'elle comprend moins de 0,5% d'eau à l'issue du procédé de fabrication, en pourcentage massique par rapport à la masse totale de ladite formulation cosmétique solide. Dans un mode de réalisation, la formulation cosmétique solide selon l'invention est caractérisée en ce qu'elle comprend moins de 0,3% d'eau à l'issue du procédé de fabrication, en pourcentage massique par rapport à la masse totale de ladite formulation cosmétique solide. Dans un mode de réalisation, la formulation cosmétique solide selon l'invention est caractérisée en ce qu'elle comprend moins de 0,2% d'eau à l'issue du procédé de fabrication, en pourcentage massique par rapport à la masse totale de ladite formulation cosmétique solide.

L'intérêt d'avoir une formulation cosmétique solide comprenant le moins possible d'eau est que la conservation de ladite formulation est beaucoup plus aisée.

Dans un mode de réalisation, la formulation cosmétique solide selon l'invention est caractérisée en ce que au moins un prébiotique est un sucre.

Dans un mode de réalisation, la formulation cosmétique solide selon l'invention est caractérisée en ce que au moins un prébiotique est un prébiotique peptidique.

Dans un mode de réalisation, la formulation cosmétique solide selon l'invention est caractérisée en ce que au moins un prébiotique est un acide linoléique conjugué.

Dans un mode de réalisation, la formulation cosmétique solide selon l'invention est caractérisée en ce que au moins un prébiotique est un acide gras polyinsaturé.

Dans un mode de réalisation, la formulation cosmétique solide selon l'invention est caractérisée en ce que au moins un prébiotique est un composé phénolique.

Dans un mode de réalisation, la formulation cosmétique solide selon l'invention est caractérisée en ce que au moins un prébiotique est un composé phytochimique.

Dans un mode de réalisation, la formulation cosmétique solide selon l'invention est caractérisée en ce qu'elle comprend entre 50 et 100 % de ladite formulation de base comprenant au moins un syndet, en pourcentage massique par rapport à la masse totale de ladite formulation cosmétique solide.

Dans un mode de réalisation, la formulation cosmétique solide selon l'invention est caractérisée en ce qu'elle comprend entre 70 et 100 % de ladite formulation de base comprenant au moins un syndet, en pourcentage massique par rapport à la masse totale de ladite formulation cosmétique solide.

Dans un mode de réalisation, la formulation cosmétique solide selon l'invention est caractérisée en ce qu'elle comprend entre 80 et 100 % de ladite formulation de base comprenant au moins un syndet, formulation de.

Dans un mode de réalisation, la formulation cosmétique solide selon l'invention est caractérisée en ce qu'elle comprend entre 85 et 96 % de ladite formulation de base comprenant au moins un syndet, formulation de.

Dans un mode de réalisation, la formulation cosmétique solide selon l'invention est caractérisée en ce qu'elle comprend entre 90 et 96 % de ladite formulation de base comprenant au moins un syndet, formulation de.

Une formulation de base de syndet comprend des tensio-actifs, des plastifiants et/ou des charges.

Des stabilisateurs ou promoteurs de mousse pourront également être ajoutés.

Dans un mode de réalisation, les tensio-actifs sont choisis dans le groupe des tensio-actifs anioniques comprenant le sodium lauryl sulfate, l'acyl isethionate, l'alkylsulfonsuccinate de sodium, le sodium cocoyl isethionate ou le disodium lauryl sulfosuccinate.

Dans un mode de réalisation, les tensio-actifs sont choisis dans le groupe des tensio-actifs cationiques comprenant le benzalkonium chloride.

Dans un mode de réalisation, les tensio-actifs sont choisis dans le groupe des tensio-actifs amphotères comprenant la cocamidopropyl bétaïne, le coco ampho acetate ou diacétate.

Dans un mode de réalisation, les plastifiants et/ou charges sont choisis parmi l'huile de ricin hydrogénée, les esters de polyols, les alcools gras éthoxylés,les acides gras, les savons, la dextrine, la talc la paraffine, la carboxymethyl cellulose, le polyprolpylene glycol, l'amidon, l'oxyde de titane.

Dans un mode de réalisation, les stabilisateurs ou promoteurs de mousse sont choisis parmi les amides de coprah comme le CMEA (coco monethanol amide).

Dans un mode de réalisation, la formulation cosmétique solide selon l'invention est caractérisée en ce que ladite formulation de base comprenant au moins un syndet est vendue sous la dénomination commerciale LANTAUROL^{®}.

Dans un mode de réalisation, la formulation cosmétique solide selon l'invention est caractérisée en ce que ladite formulation de base comprenant au moins un syndet a la composition qualitative suivante : sodium cocoyl isethionate, disodium lauryl sulfosuccinate, maltodextrine, acide stéarique, eau, huile hydrogénée de ricin, alcool cétylique, acide citrique, oxyde de titane et commercialisée sous le nom de LANTAUROL MI HS 27-21 SP^{®}.

Dans un mode de réalisation, la formulation cosmétique solide selon l'invention est caractérisée en ce que ladite formulation de base comprenant au moins un syndet a la composition qualitative suivante : sodium cocoyl isethionate, disodium lauryl sulfosuccinate, maltodextrine, acide stéarique, eau, huile hydrogénée de ricin, alcool cétylique, acide citrique, oxyde de titane.

Dans un mode de réalisation, la formulation cosmétique solide selon l'invention est caractérisée en ce que ladite formulation de base comprenant au moins un syndet comprend entre 30 et 75 % de syndet, en pourcentage massique par rapport à la masse totale de ladite formulation cosmétique solide.

Dans un mode de réalisation, la formulation cosmétique solide selon l'invention est caractérisée en ce que ladite formulation de base comprenant au moins un syndet comprend entre 35 et 55 % de syndet, en pourcentage massique par rapport à la masse totale de ladite formulation cosmétique solide.

Dans un mode de réalisation, la formulation cosmétique solide selon l'invention est caractérisée en ce que ladite formulation de base comprenant au moins un syndet comprend entre 40 et 50 % de syndet, en pourcentage massique par rapport à la masse totale de ladite formulation cosmétique solide.

Dans un mode de réalisation, la formulation cosmétique solide selon l'invention est caractérisée en ce qu'elle comprend entre 0,5 et 5 % de prébiotique, en pourcentage massique par rapport à la masse totale de ladite formulation cosmétique solide.

Dans un mode de réalisation, la formulation cosmétique solide selon l'invention est caractérisée en ce qu'elle comprend entre 1 et 4 % de prébiotique, en pourcentage massique par rapport à la masse totale de ladite formulation cosmétique solide.

Dans un mode de réalisation, la formulation cosmétique solide selon l'invention est caractérisée en ce qu'elle comprend entre 1,5 et 3 % de prébiotique, en pourcentage massique par rapport à la masse totale de ladite formulation cosmétique solide.

Dans un mode de réalisation, la formulation cosmétique solide selon l'invention est caractérisée en ce que lesdits prébiotiques sont choisis dans le groupe comprenant l'inuline (commercialisé sous le nom INUTEC H25P^{®}), l'alphaglucane oligosaccharide (commercialisé sous le nom BIOECOLIA^{®}), les transgalacto-oligosaccharides (TOS), les fructo-oligosaccarides (FOS), les galacto-oligosaccharides (GOS), et leurs mélanges.

Dans un mode de réalisation, la formulation cosmétique solide selon l'invention est caractérisée en ce que lesdits prébiotiques sont choisis dans le groupe comprenant l'inuline, l'alphaglucane oligosaccharide, les transgalacto-oligosaccharides (TOS), les fructo-oligosaccarides (FOS), les galacto-oligosaccharides (GOS), et leurs mélanges.

Dans un mode de réalisation, la formulation cosmétique solide selon l'invention est caractérisée en ce que lesdits prébiotiques sont choisis dans le groupe comprenant l'inuline, l'alphaglucane oligosaccharide, et leurs mélanges.

Dans un mode de réalisation, la formulation cosmétique solide selon l'invention est caractérisée en ce que lesdits prébiotiques sont un mélange d'inuline et d'alphaglucane oligosaccharide.

La formulation cosmétique solide selon l'invention est caractérisée en ce qu'elle comprend au moins 2 prébiotiques différents.

Dans un mode de réalisation, la formulation cosmétique solide selon l'invention est caractérisée en ce qu'elle comprend au moins 3 prébiotiques différents.

Dans un mode de réalisation, la formulation cosmétique solide selon l'invention est caractérisée en ce qu'elle comprend au moins 4 prébiotiques différents.

Dans un mode de réalisation, la formulation cosmétique solide selon l'invention est caractérisée en ce qu'elle comprend entre 0,01 et 2 % desdits postbiotiques, en pourcentage massique de ladite formulation cosmétique solide.

Dans un mode de réalisation, la formulation cosmétique solide selon l'invention est caractérisée en ce qu'elle comprend entre 0,1 et 1 % desdits postbiotiques, en pourcentage massique de ladite formulation cosmétique solide.

Dans un mode de réalisation, la formulation cosmétique solide selon l'invention est caractérisée en ce qu'elle comprend entre 0,3 et 0,6 % desdits postbiotiques, en pourcentage massique de ladite formulation cosmétique solide.

La formulation cosmétique solide selon l'invention comprend au moins deux postbiotiques différents dont l'acide lactique et l'arginine.

Dans un mode de réalisation, la formulation cosmétique solide selon l'invention est caractérisée en ce que au moins un autre postbiotique est choisi dans le groupe comprenant l'acide acétique, l'acide butyrique, l'acide propionique, les produits de fermentation, les acides gras à chaine courte, et leurs mélanges.

Dans un mode de réalisation, la formulation cosmétique solide selon l'invention est caractérisée en ce que au moins un autre postbiotique est choisi dans le groupe comprenant l'acide lactique, l'acide acétique, l'acide butyrique, l'acide propionique, et leurs mélanges.

Dans un mode de réalisation, la formulation cosmétique solide selon l'invention est caractérisée en ce qu'elle comprend entre 0,01 et 2 % dudit acide lactique, en pourcentage massique de ladite formulation cosmétique solide.

Dans un mode de réalisation, la formulation cosmétique solide selon l'invention est caractérisée en ce qu'elle comprend entre 0,1 et 1 % dudit acide lactique, en pourcentage massique de ladite formulation cosmétique solide.

Dans un mode de réalisation, la formulation cosmétique solide selon l'invention est caractérisée en ce qu'elle comprend entre 0,3 et 0,6 % dudit acide lactique, en pourcentage massique de ladite formulation cosmétique solide.

Dans un mode de réalisation, la formulation cosmétique solide selon l'invention est caractérisée en ce que le rapport massique entre la base comprenant au moins un syndet et le prébiotique est compris entre 30 et 100.

Dans un mode de réalisation, la formulation cosmétique solide selon l'invention est caractérisée en ce que le rapport massique entre la base comprenant au moins un syndet et le prébiotique est compris entre 30 et 80.

Dans un mode de réalisation, la formulation cosmétique solide selon l'invention est caractérisée en ce que le rapport massique entre la base comprenant au moins un syndet et le prébiotique est compris entre 40 et 50.

Dans un mode de réalisation, la formulation cosmétique solide selon l'invention est caractérisée en ce que le rapport massique entre la base comprenant au moins un syndet et le postbiotique est compris entre 50 et 250.

Dans un mode de réalisation, la formulation cosmétique solide selon l'invention est caractérisée en ce que le rapport massique entre la base comprenant au moins un syndet et le postbiotique est compris entre 75 et 200.

Dans un mode de réalisation, la formulation cosmétique solide selon l'invention est caractérisée en ce que le rapport massique entre la base comprenant au moins un syndet et le postbiotique est compris entre 100 et 150.

Dans un mode de réalisation, la formulation cosmétique solide selon l'invention est caractérisée en ce que le rapport massique entre le prébiotique et ledit le postbiotique est compris entre 1 et 5.

Dans un mode de réalisation, la formulation cosmétique solide selon l'invention est caractérisée en ce que le rapport massique entre le prébiotique et le postbiotique est compris entre 1,5 et 4.

Dans un mode de réalisation, la formulation cosmétique solide selon l'invention est caractérisée en ce que le rapport massique entre le prébiotique et le postbiotique est compris entre 2 et 3.

La formulation cosmétique solide selon l'invention est caractérisée en ce qu'elle comprend au moins 2 postbiotiques différents dont l'acide lactique et l'arginine.

Dans un mode de réalisation, la formulation cosmétique solide selon l'invention est caractérisée en ce qu'elle comprend au moins 3 postbiotiques différents.

Dans un mode de réalisation, la formulation cosmétique solide selon l'invention est caractérisée en ce qu'elle comprend au moins 4 postbiotiques différents.

La formulation cosmétique solide selon l'invention est caractérisée en ce que ledit acide lactique est combiné à l'arginine, comme par exemple dans la composition commerciale AH CARE L65^{®}.

En effet, la présence d'arginine permet de ne pas piquer les peaux les plus sensibles. De plus, la combinaison de l'acide lactique sur l'arginine permet d'obtenir une libération prolongée de l'acide lactique.

Dans un mode de réalisation, la formulation cosmétique solide selon l'invention est caractérisée en ce qu'elle comprend en outre un parfum.

Dans un mode de réalisation, la formulation cosmétique solide selon l'invention est caractérisée en ce qu'elle comprend en outre un parfum aux agrumes.

Dans un mode de réalisation, la formulation cosmétique solide selon l'invention est caractérisée en ce qu'elle comprend en outre le parfum fraicheur d'agrumes.

Dans un mode de réalisation, la formulation cosmétique solide selon l'invention est caractérisée en ce qu'elle comprend entre 0,1 et 1,5 % dudit parfum, en pourcentage massique de ladite formulation cosmétique solide.

Dans un mode de réalisation, la formulation cosmétique solide selon l'invention est caractérisée en ce qu'elle comprend entre 0,1 et 1 % dudit parfum, en pourcentage massique de ladite formulation cosmétique solide.

Dans un mode de réalisation, la formulation cosmétique solide selon l'invention est caractérisée en ce qu'elle comprend entre 0,3 et 0,8 % dudit parfum, en pourcentage massique de ladite formulation cosmétique solide.

Dans un mode de réalisation, la formulation cosmétique solide selon l'invention est caractérisée en ce que ladite composition cosmétique solide a une masse comprise entre 10 et 1000 grammes.

Dans un mode de réalisation, la formulation cosmétique solide selon l'invention est caractérisée en ce que ladite composition cosmétique solide a une masse comprise entre 50 et 500 grammes.

Dans un mode de réalisation, la formulation cosmétique solide selon l'invention est caractérisée en ce que ladite composition cosmétique solide a une masse comprise entre 50 et 250 grammes.

Dans un mode de réalisation, la formulation cosmétique solide selon l'invention est caractérisée en ce que ladite composition cosmétique solide a une masse comprise entre 75 et 130 grammes.

Dans un mode de réalisation, la formulation cosmétique solide selon l'invention est caractérisée en ce que ladite composition cosmétique solide a une masse de 125 grammes.

Dans un mode de réalisation, la formulation cosmétique solide selon l'invention est caractérisée en ce que ladite composition cosmétique solide a une masse de 100 grammes.

Dans un mode de réalisation, la formulation cosmétique solide selon l'invention est caractérisée en ce que ladite composition cosmétique solide a un volume compris entre 7 et 1200 cm3.

Dans un mode de réalisation, la formulation cosmétique solide selon l'invention est caractérisée en ce que ladite composition cosmétique solide a un volume compris entre 50 et 600 cm3.

Dans un mode de réalisation, la formulation cosmétique solide selon l'invention est caractérisée en ce que ladite composition cosmétique solide a un volume compris entre 100 et 300 cm3.

Dans un mode de réalisation, la formulation cosmétique solide selon l'invention est caractérisée en ce que ladite composition cosmétique solide a un volume compris entre 115 et 130 cm3.

Dans un mode de réalisation, la formulation cosmétique solide selon l'invention est caractérisée en ce que ladite composition cosmétique solide a une forme choisie dans le groupe comprenant le parallélépipède, le cylindre, le prisme, et le cube.

Dans un mode de réalisation, la formulation cosmétique solide selon l'invention est caractérisée en ce que ladite composition cosmétique solide a une forme de parallélépipède.

Dans un mode de réalisation, la formulation cosmétique solide selon l'invention est caractérisée en ce que ladite composition cosmétique solide a une forme de parallélépipède rectangle.

Dans un mode de réalisation, la formulation cosmétique solide selon l'invention est caractérisée en ce que ladite composition cosmétique solide a une forme de parallélépipède rectangle ayant les dimensions suivantes : 70 x 70 x 24 mm.

Dans un mode de réalisation, la formulation cosmétique solide selon l'invention est caractérisée en ce que ladite composition cosmétique solide a une forme de parallélépipède rectangle ayant les dimensions suivantes : 110 x 110 x 35 mm.

Dans un mode de réalisation, la formulation cosmétique solide selon l'invention est caractérisée en ce que ladite composition cosmétique solide a une forme de parallélépipède rectangle ayant les dimensions suivantes : 150 x 80 x 50 mm.

Dans un mode de réalisation, la formulation cosmétique solide selon l'invention est caractérisée en ce que ladite composition cosmétique solide a une forme de cube.

Dans un mode de réalisation, la formulation cosmétique solide selon l'invention est caractérisée en ce que ladite composition cosmétique solide présente une zone d'information.

Dans un mode de réalisation, la formulation cosmétique solide selon l'invention est caractérisée en ce que l'activité de l'eau est inférieur à 0,90 à 20°C.

Dans un mode de réalisation, la formulation cosmétique solide selon l'invention est caractérisée en ce que l'activité de l'eau est inférieur à 0,85 à 20°C.

Dans un mode de réalisation, la formulation cosmétique solide selon l'invention est caractérisée en ce qu'elle ne comprend pas de savon.

Dans un mode de réalisation, la formulation cosmétique solide selon l'invention est caractérisée en ce qu'elle comprend un mélange de base comprenant au moins un syndet et de savon.

La formulation cosmétique solide selon l'invention est caractérisée en ce que sa fabrication ne comprend aucune étape de moulage.

Dans un mode de réalisation, la formulation cosmétique solide selon l'invention est une formulation cosmétique solide nettoyante utilisée avec de l'eau.

L'invention concerne également un procédé de fabrication d'une formulation cosmétique solide selon l'invention, caractérisé en ce qu'il ne comprend aucune étape de moulage.

L'invention concerne également un procédé de fabrication d'une formulation cosmétique solide selon l'invention, caractérisé en ce qu'il ne comprend aucune étape de démoulage.

L'invention concerne également un procédé de fabrication d'une formulation cosmétique solide selon l'invention, caractérisé en ce que ladite formulation est obtenue à l'issue d'une étape de découpe.

L'invention concerne également un procédé de fabrication d'une formulation cosmétique solide selon l'invention, caractérisé en ce que ladite formulation est obtenue à l'issue d'une étape de découpe, et en ce qu'il ne comprend aucune étape de moulage.

L'invention concerne également l'utilisation d'une formulation cosmétique solide selon l'invention, notamment tel que décrit ci-dessous. Ces utilisations sont de l'ordre cosmétique, non-thérapeutique.

L'invention concerne également l'utilisation d'une formulation cosmétique solide selon l'invention, pour le lavage du tégument (peau, phanères).

L'invention concerne également l'utilisation d'une formulation cosmétique solide selon l'invention, pour le lavage de la peau.

L'invention concerne également l'utilisation d'une formulation cosmétique solide selon l'invention, pour le lavage des phanères.

L'invention concerne également l'utilisation d'une formulation cosmétique solide selon l'invention, pour le lavage des muqueuses.

L'invention concerne également l'utilisation d'une formulation cosmétique solide selon l'invention, pour le lavage des muqueuses génitales.

L'invention concerne également l'utilisation d'une formulation cosmétique solide selon l'invention, pour l'hygiène intime.

L'invention concerne également l'utilisation d'une formulation cosmétique solide selon l'invention, pour nettoyer une peau délicate.

L'invention concerne également l'utilisation d'une formulation cosmétique solide selon l'invention, pour adoucir la peau.

L'invention concerne également l'utilisation d'une formulation cosmétique solide selon l'invention, pour apaiser.

L'invention concerne également l'utilisation d'une formulation cosmétique solide selon l'invention, pour restaurer la barrière cutanée.

L'invention concerne également l'utilisation d'une formulation cosmétique solide selon l'invention, pour prévenir du dessèchement de la peau.

L'invention concerne également l'utilisation d'une formulation cosmétique solide selon l'invention, pour diminuer les érythèmes.

L'invention concerne également l'utilisation d'une formulation cosmétique solide selon l'invention, pour diminuer la sécheresse cutanée.

L'invention concerne également l'utilisation d'une formulation cosmétique solide selon l'invention, pour diminuer la desquamation.

L'invention concerne également l'utilisation d'une formulation cosmétique solide selon l'invention, pour diminuer l'hyperkératose.

Les avantages de la formulation cosmétique solide selon l'invention sont décrits ci-dessous.

En outre, l'utilisation de syndet permet la préservation de la faune et de la flore.

### EXEMPLES

### Exemple 1 : Formulations cosmétiques solides selon l'invention

Une formulation cosmétique solide selon l'invention a été préparée en mettant en œuvre les matières premières suivantes :

| **Formulation cosmétique solide selon l'invention 1** | | | |
|---|---|---|---|
| Fonctions | INCI | Nom commercial | Pourcentages massiques (%) |
| Base comprenant au moins un syndet | sodium cocoyl isethionate disodium lauryl sulfosuccinate maltodextrine acide stéarique eau huile hydrogénée de ricin alcool cétylique acide citrique dioxyde de titane | LANTAUROL^{®} MI HS 27-21 SP | 95,76 |
| Prébiotique | Inuline eau | INUTEC H25P^{®} | 1,00 |
| Prébiotique | Alpha-glucan oligosaccharide eau | BIOECOLIA^{®} | 1,00 |
| Solvant | eau | | 1,00 |
| Postbiotique | acide lactique eau Arginine | AH CARE L65^{®} | 0,74 |
| Parfum | Parfum | PARFUM FRAICHEUR D'AGRUMES 1519796 | 0,50 |

Le procédé de préparation est le suivant : dissolution des prébiotiques dans le postbiotique, ajout de 1% d'eau, puis ce pré-mélange à 55°C est introduit dans la formulation de base comprenant au moins un syndet.

Une fois le procédé achevé, le pH et la teneur en eau sont mesurés. Le pH est de 5,8, et la teneur en eau est inférieure à 1% (pourcentage massique).

Une seconde formulation cosmétique solide selon l'invention a été préparée en mettant en œuvre les matières premières suivantes :

| **Formulation cosmétique solide selon l'invention 2** | | | |
|---|---|---|---|
| Fonctions | INCI | Nom commercial | Pourcentages massiques (%) |
| Base comprenant au moins un syndet | sodium cocoyl isethionate disodium lauryl sulfosuccinate maltodextrine acide stéarique eau huile hydrogénée de ricin alcool cétylique acide citrique dioxyde de titane | LANTAUROL^{®} MI HS 27-21 SP | 94,76 |
| Agent de texture | glycerine | | 1,00 |
| Prébiotique | Inuline eau | INUTEC H25P^{®} | 1,00 |
| Prébiotique | Alpha-glucan oligosaccharide eau | BIOECOLIA^{®} | 1,00 |
| Solvant | eau | | 1,00 |
| Postbiotique | acide lactique eau Arqinine | AH CARE L65^{®} | 0,74 |
| Parfum | Parfum | PARFUM FRAICHEUR D'AGRUMES 1519796 | 0,50 |

A titre d'information, la composition LANTAUROL^{®} MI HS 27-21 SP comprend environ 8% en masse d'eau.

Le procédé de préparation est le suivant :
- introduire dans un mélangeur la base comprenant au moins un syndet ;
- introduire dans le mélangeur la glycérine ;
- solubiliser les prébiotiques, le postbiotique dans l'eau puis chauffer à 55°C sous agitation, puis vérifier l'homogénéité ;
- introduire dans le mélangeur les prébiotiques, le postbiotique et l'eau ;
- introduire dans le mélangeur le parfum ;
- broyer ;
- extruder ;
- découper.

A la sortie de l'extrudeuse, la température du mélange est d'environ 80 à 90°C.

Au cours du procédé, une grande partie de l'eau s'évapore, et le pourcentage massique d'eau dans la formulation solide selon l'invention en fin de procédé est par exemple d'environ 0,01 à 1% par rapport à la masse totale de ladite formulation.

### Exemple 2 : Evaluation de la tolérance cutanée (symptomatologie clinique et fonctionnelle) et de l'efficacité perçue d'une composition cosmétique solide selon l'invention.

### 1. Présentation

La tolérance cutanée (symptomatologie clinique et fonctionnelle) et l'efficacité perçue d'une composition cosmétique solide selon l'invention ont été évaluées.

La formulation cosmétique solide selon l'invention est la formulation 1 de l'exemple 1.

Durant 28 jours, il a été demandé à 22 volontaires (femmes ; âge moyen de 56 ans (plus ou moins 12 ans), compris entre 29 et 70 ans) d'appliquer une formulation cosmétique solide selon l'invention 2 fois par jour au niveau du visage humide (matin et soir) et 1 fois par jour au niveau du corps humide.

L'évaluation de formulation cosmétique solide selon l'invention a été faite au cours de 2 visites en début et fin de test (à J0 et à J28) chez un dermatologue, ainsi que par auto-évaluation.

### 2. Tolérance : symptomatologie clinique dermatologique

Le dermatologue a réalisé une observation de l'état cutané de chaque volontaire au niveau du visage, des avant-bras et des mollets à J0 et J28 en prenant en compte divers signes cliniques.

L'importance des divers signes cliniques observés sur les différentes zones sera cotée de 0 (absence de signe) à 12 (signe sévère).

Les signes cliniques cutanés observés par le dermatologue étaient les suivants : sécheresse, desquamation et hyperkératose.

L'ensemble des résultats sont donnés dans le Tableau 1 ci-dessous :

**Tableau 1 : symptomatologie clinique dermatologique**

| | Sécheresse | | Desquamation | | Hyperkératose | |
|---|---|---|---|---|---|---|
| Volontaire | J0 | J28 | J0 | J28 | J0 | J28 |
| Volontaire 1 | 11 | 12 | 10 | 9 | 0 | 0 |
| Volontaire 2 | 11 | 10 | 11 | 10 | 0 | 0 |
| Volontaire 3 | 9 | 7 | 7 | 5 | 0 | 0 |
| Volontaire 4 | 6 | 7 | 4 | 6 | 0 | 0 |
| Volontaire 5 | 6 | 4 | 5 | 4 | 0 | 0 |
| Volontaire 6 | 2 | 2 | 2 | 2 | 0 | 0 |
| Volontaire 7 | 9 | 12 | 8 | 10 | 3 | 0 |
| Volontaire 8 | 6 | 4 | 4 | 4 | 0 | 0 |
| Volontaire 9 | 2 | 2 | 2 | 2 | 0 | 0 |
| Volontaire 10 | 0 | 2 | 0 | 2 | 0 | 0 |
| Volontaire 11 | 2 | 6 | 2 | 6 | 0 | 0 |
| Volontaire 12 | 7 | 7 | 6 | 6 | 0 | 0 |
| Volontaire 13 | 4 | 4 | 3 | 3 | 3 | 0 |
| Volontaire 14 | 0 | 0 | 0 | 0 | 0 | 0 |
| Volontaire 15 | 8 | 6 | 7 | 6 | 0 | 0 |
| Volontaire 16 | 10 | 9 | 9 | 8 | 0 | 0 |
| Volontaire 17 | 12 | 12 | 12 | 10 | 8 | 5 |
| Volontaire 18 | 3 | 3 | 2 | 2 | 0 | 0 |
| Volontaire 19 | 2 | 0 | 2 | 0 | 0 | 0 |
| Volontaire 20 | 0 | 0 | 0 | 0 | 0 | 0 |
| Volontaire 21 | 9 | 7 | 9 | 7 | 0 | 0 |
| Volontaire 22 | 6 | 4 | 6 | 4 | 0 | 0 |
| *Moyenne* | *5,682* | *5,455* | *5,045* | *4,818* | *0,636* | *0,227* |
| *Ecart-type* | *3,884* | *3,851* | *3,697* | *3,275* | *1,866* | *1,066* |

Les données de la symptomatologie clinique dermatologique étaient ensuite traitées par le test non paramétrique des rangs de Wilcoxon avec pour hypothèse nulle H0 : (A) = (B), et pour hypothèse alternative H1 : (A) ≠ (B).
(A) : symptomatologie clinique à J0
(B) : symptomatologie clinique à J28

Si le seuil de significativité obtenu (p) était < 0,05, on rejetait l'hypothèse nulle H0. On acceptait alors une hypothèse alternative H1 d'une différence significative entre la symptomatologie clinique observée à J28 et celle à J0.

Le logiciel utilisé était XLSTAT 2017.

Le tableau 2 ci-dessous regroupe les résultats du test de Wilcoxon de comparaison des scores cliniques relevés par le dermatologue à J0 et J28 :

**Tableau 2 : résultats du test de Wilcoxon de comparaison des scores cliniques**

| Comparaison J28/J0 | Sécheresse | Desquamation | Hyperkératose |
|---|---|---|---|
| Seuil de significativité | p = 0.440 | p = 0.519 | p = 0.083 |

Le seuil de significativité obtenu (p) est > 0,050 pour tous les signes cliniques observés.

### Conclusion : la formulation cosmétique solide selon l'invention est bénéfique en ce qui concerne les signes cliniques étudiés.

### 3. Tolérance : symptomatologie fonctionnelle

La symptomatologie fonctionnelle globale était évaluée par le volontaire selon l'échelle suivante : excellente, très bonne, bonne, assez bonne, moyenne, assez mauvaise, mauvaise.

Les 22 volontaires présents aux deux visites médicales (J0 et J28) n'ont ressenti aucun désagrément en cours d'étude. Pour ces 22 volontaires (soit 100% du panel), la tolérance individuelle fonctionnelle a été considérée comme étant « excellente ».

### Conclusion : la formulation cosmétique solide selon l'invention a une tolérance individuelle fonctionnelle excellente.

### 4. Efficacité perçue et acceptabilité

En fin de test (J28), les volontaires complétaient des questionnaires concernant l'efficacité perçue et l'acceptabilité du produit à l'essai. Les volontaires répondaient à des questions selon l'échelle en 4 points : Pas du tout d'accord ; Plutôt pas d'accord ; Plutôt d'accord ; Tout à fait d'accord.

Les résultats sont donnés dans le tableau 3 ci-dessous :

**Tableau 3 : efficacité perçue et acceptabilité**

| | **Pas du tout d'accord** | **Plutôt pas d'accord** | **Plutôt d'accord** | **Tout à fait d'accord** |
|---|---|---|---|---|
| La texture du produit est crémeuse | 0,0% | 8,7% | 47,8% | 43,5% |
| La mousse est délicate | 0,0% | 17,4% | 47,8% | 34,8% |
| La formule est ultra douce | 0,0% | 4,3% | 47,8% | 47,9% |
| Le produit nettoie parfaitement les peaux délicates | 0,0% | 4,3% | 52,2% | 43,5% |
| Le produit nettoie sans agresser | 4,3% | 8,7% | 52,2% | 34,8% |
| Le produit nettoie en douceur | 0,0% | 4,3% | 56,5% | 39,2% |
| Le produit adoucit la peau | 4,3% | 21,7% | 39,1% | 34,9% |
| Le produit apaise instantanément | 4,3% | 26,1% | 47,8% | 21,8% |
| Le produit apaise durablement | 4,3% | 30,4% | 47,8% | 17,5% |
| Le produit aide à restaurer la barrière cutanée | 8,7% | 26,1% | 39,1% | 26,1% |
| Le produit protège la peau | 0,0% | 21,7% | 52,2% | 26,1% |
| Le produit nourrit la peau | 4,3% | 13,0% | 52,2% | 30,5% |
| Le produit prévient du dessèchement de la peau | 8,7% | 30,4% | 39,1% | 21,8% |
| Le produit respecte le film hydrolipidique de la peau | 4,3% | 8,7% | 60,9% | 26,1% |
| Le produit soulage des tiraillements | 8,7% | 34,8% | 34,8% | 21,7% |
| Le produit calme les sensations d'inconfort | 4,3% | 39,1% | 43,5% | 13,0% |
| La peau est infiniment douce | 8,7% | 26,1% | 34,8% | 30,4% |
| La peau est propre | 0,0% | 0,0% | 65,2% | 34,8% |
| La peau est confortable | 4,3% | 8,7% | 56,5% | 30,5% |
| La peau retrouve sa souplesse | 8,7% | 26,1% | 34,8% | 30,4% |
| Vous êtes satisfait(e) du produit | 4,3% | 21,7% | 39,1% | 34,9% |
| Vous achèteriez ce produit s'il était sur le marché à un prix raisonnable | 8,7% | 26,1% | 30,4% | 34,8% |

**Conclusion** : **globalement, la formulation cosmétique solide selon l'invention présente une efficacité et une acceptabilité satisfaisantes.**

## Revendications

1. Formulation cosmétique solide, comprenant :
- une formulation de base comprenant au moins un syndet ;
- au moins deux prébiotiques différents ;
- au moins deux postbiotiques différents dont l'acide lactique et l'arginine ;
**caractérisée en ce que** sa fabrication ne comprend aucune étape de moulage.

2. Formulation cosmétique solide selon l'une quelconque des revendications précédentes, **caractérisée en ce que** le pH de ladite formulation cosmétique solide cosmétique est inférieur à 6.

3. Formulation cosmétique solide selon l'une quelconque des revendications précédentes, **caractérisée en ce qu'**elle comprend moins de 5% d'eau, en pourcentage massique par rapport à la masse totale de ladite formulation cosmétique solide.

4. Formulation cosmétique solide selon l'une quelconque des revendications précédentes, **caractérisée en ce qu'**elle comprend entre 50 et 100 % de ladite base comprenant au moins un syndet, en pourcentage massique par rapport à la masse totale de ladite formulation cosmétique solide.

5. Formulation cosmétique solide selon l'une quelconque des revendications précédentes, **caractérisée en ce qu'**elle comprend entre 0,5 et 5 % desdits prébiotiques, en pourcentage massique par rapport à la masse totale de ladite formulation cosmétique solide.

6. Formulation cosmétique solide selon l'une quelconque des revendications précédentes, **caractérisée en ce que** au moins un prébiotique est choisis dans le groupe comprenant l'inuline, l'alphaglucane oligosaccharide, les transgalacto-oligosaccharides (TOS), les fructo-oligosaccarides (FOS), les galacto-oligosaccharides (GOS), et leurs mélanges.

7. Formulation cosmétique solide selon l'une quelconque des revendications précédentes, **caractérisée en ce qu'**elle comprend entre 0,01 et 2 % desdits postbiotiques, en pourcentage massique par rapport à la masse totale de ladite formulation cosmétique solide.

## Patentansprüche

1. Feste kosmetische Formulierung umfassend:
- eine Basisformulierung umfassend wenigstens ein Syndet,
- mindestens zwei verschiedene Präbiotika,
- mindestens zwei verschiedene Postbiotika, darunter Milchsäure und Arginin,
**dadurch gekennzeichnet, dass** deren Herstellung keinen Formungsschritt umfasst.

2. Feste kosmetische Formulierung nach einem der vorangehenden Ansprüche, **dadurch gekennzeichnet, dass** der pH der festen kosmetischen Formulierung weniger als 6 beträgt.

3. Feste kosmetische Formulierung nach einem der vorangehenden Ansprüche, **dadurch gekennzeichnet, dass** sie mindestens 5 Gew.- % Wasser bezogen auf das Gesamtgewicht der festen kosmetischen Formulierung umfasst.

4. Feste kosmetische Formulierung nach einem der vorangehenden Ansprüche, **dadurch gekennzeichnet, dass** sie zwischen 50 und 100 Gew.-% an der mindestens ein Syndet umfassenden Basis bezogen auf das Gesamtgewicht der festen kosmetischen Formulierung umfasst.

5. Feste kosmetische Formulierung nach einem der vorangehenden Ansprüche, **dadurch gekennzeichnet, dass** sie zwischen 0,5 und 5,0 Gew.-% an den Präbiotika bezogen auf das Gesamtgewicht der festen kosmetischen Formulierung umfasst.

6. Feste kosmetische Formulierung nach einem der vorangehenden Ansprüche, **dadurch gekennzeichnet, dass** zumindest ein Präbiotikum ausgewählt ist aus der Gruppe umfassend Inulin, Alphaglucan-Oligosaccharid, Transgalacto-Oligosaccharide (TOS), Fructo-Oligosaccharide, Galakto-Oligosaccharide (GOS) und Mischungen davon.

7. Feste kosmetische Formulierung nach einem der vorangehenden Ansprüche, **dadurch gekennzeichnet, dass** sie zwischen 0,01 und 2 Gew.-% an den Postbiotika bezogen auf das Gesamtgewicht der festen kosmetischen Formulierung umfasst.

## Claims

1. Solid cosmetic formulation, comprising:
- a basic formulation comprising at least one syndet;
- at least two different prebiotics;
- at least two different postbiotics including lactic acid and arginine;
**characterised in that** its manufacture does not include any moulding step.

2. Solid cosmetic formulation according to any one of the preceding claims, **characterised in that** the pH of said solid cosmetic formulation is less than 6.

3. Solid cosmetic formulation according to any one of the preceding claims, **characterised in that** it comprises less than 5% water, as a mass percentage relative to the total mass of said solid cosmetic formulation.

4. Solid cosmetic formulation according to any one of the preceding claims, **characterised in that** it comprises between 50 and 100% of said base comprising at least one syndet, as a mass percentage relative to the total mass of said solid cosmetic formulation.

5. cosmetic formulation according to any one of the preceding claims, **characterised in that** it comprises between 0.5 and 5% of said prebiotics, as a mass percentage relative to the total mass of said solid cosmetic formulation.

6. Solid cosmetic formulation according to any one of the preceding claims, **characterised in that** at least one prebiotic is chosen from the group comprising inulin, alphaglucan oligosaccharide, trans galacto-oligosaccharides (TOS), fructo-oligosaccharides (FOS), galacto-oligosaccharides (GOS), and mixtures thereof.

7. Solid cosmetic formulation according to any one of the preceding claims, **characterised in that** it comprises between 0.01 and 2% of said postbiotics, as a mass percentage relative to the total mass of said solid cosmetic formulation.
